# FASCICULE DE BREVET EUROPEEN

(11) **EP 1 129 676 B1**
(45) Date de publication et mention de la délivrance du brevet: **11.05.2005**
(21) Numéro de dépôt: 01400440.2
(22) Date de dépôt: 20.02.2001
(51) Int. Cl.: A61F 2/38

(54) **Composant fémoral d'une prothèse du genou à trois rayons de courbure**
Femorale Komponente einer Knieprothese mit drei Krümmungsradien
Femoral piece of a knee prosthesis with three radii of curvature

(30) Priorité: 24.02.2000 FR 0002312
(43) Date de publication de la demande: 05.09.2001
(73) Titulaire: Aesculap AG & Co. KG, 78532 Tuttlingen (DE)
(72) Inventeur: Biegun, Jean-François, 52000 Chaumont (FR); Marceaux, Pascal, 52000 Chaumont (FR)
(74) Mandataire: Eidelsberg, Olivier

(56) Documents cités:
- EP-A- 0 765 645
- US-A- 3 964 106
- US-A- 5 011 496
- US-A- 5 824 100
- US-A- 6 013 103

## Description

La présente invention concerne des parties fémorales d'une prothèse de genou. Elle concerne notamment des parties fémorales d'une prothèse du type à trois compartiments, à savoir deux parties formant condyles qui sont séparées par une surface de trochlée.

Ces parties fémorales de prothèse sont destinées à fonctionner en coopération avec un insert, souvent en un matériau tel que le polyéthylène, posé sur une plaque tibiale d'une partie tibiale, la coopération s'effectuant par l'intermédiaire de surfaces supérieures concaves de l'insert sur lesquelles roulent et glissent les surfaces extérieures des condyles de la partie fémorale.

On connaît déjà de l'art antérieur, et notamment de la demande internationale WO92/03108 au nom de British Technology Group PLC, une partie fémorale de prothèse de ce genre. Suivant une coupe transversale longitudinale, c'est-à-dire suivant un plan parallèle à l'axe longitudinal du fémur et à l'axe longitudinal du tibia, en position fléchie du genou, cette partie fémorale de prothèse présente deux arcs de cercle, à savoir un premier arc de cercle correspondant à la surface de trochlée, définissant ce que l'on appelle le trajet trochléen. Nous désignerons par la suite ce rayon par la référence R1. Le deuxième arc de cercle de cette coupe transversale dans le plan longitudinal correspond aux deux condyles. Cet arc de cercle sera désigné par la suite sous le terme R2.

Suivant l'art antérieur, le rayon R1 est légèrement supérieur au rayon R2 à savoir en général suivant un rapport de 1,2. Ce type de parties fémorales, lorsqu'elles sont utilisées en coopération avec des surfaces concaves supérieures d'insert tibial de forme sphérique ou circulaire cylindrique correspondantes, assurent une congruence parfaite entre les surfaces des condyles et les surfaces de l'insert tibial sur tout le domaine de flexion à savoir de 0 à 130°. Cependant, ce type de prothèse du genou de l'art antérieur présente l'inconvénient que les contraintes auxquelles sont soumis les ligaments, et notamment le ligament croisé postérieur, dans le domaine de flexion compris entre 80 ou 90° et 130° sont très importantes.

US-A-6 013 103 et EP 0 765 645 décrivent des prothèses comme définies au préambule de la revendication 1.

La congruence y est totale avec la partie tibiale de sorte que les contraintes subies par les prothèses aux grandes flexions sont très importantes, ce qui nuit à leur longévité.

La présente invention vise à pallier cet inconvénient en proposant une partie fémorale de prothèse qui permet une diminution des contraintes appliquées aux ligaments dans le domaine de flexion entre 80 à 90° et 130°.

Suivant l'invention, la partie fémorale d'une prothèse de genou est telle que définie à la revendication 1.

Des perfectionnements sont définies aux sous-revendications.

On obtient ainsi une congruence parfaite avec des surfaces concaves d'insert tibial ayant un rayon de courbure à peu près égal au rayon de courbure de l'arc de cercle intermédiaire sur un domaine de flexion de 80° à 90°, mais en revanche, pour une grande flexion c'est-à-dire au-delà de 80° ou 90°, la congruence diminue de sorte qu'un certain jeu apparaît entre la partie fémorale et l'insert tibial, ce jeu permettant de relâcher les contraintes auxquelles sont soumis les ligaments, notamment le ligament croisé postérieur, de sorte que les prothèses de genou comportant une partie fémorale de ce genre peuvent être utilisées sans porter atteinte sur le long terme au ligament croisé postérieur.

Suivant un perfectionnement de l'invention, le rapport de l'arc de cercle intermédiaire sur l'arc de cercle d'extrémité est compris entre 1 et 2,5, de préférence entre 1,2 et 1,8.

L'invention vise également une protection du genou comportant une partie fémorale telle que définie précédemment et un insert tibial posé sur une plaque tibiale, l'insert tibial ayant une surface concave supérieure coopérant avec la surface extérieure des condyles, le rayon de courbure de la surface extérieure de l'insert étant sensiblement égal au rayon de courbure de l'arc de cercle intermédiaire.

Un mode de réalisation donné uniquement à titre d'exemple est décrit aux dessins, dans lesquels :
La figure 1 est une vue en coupe longitudinale, c'est-à-dire dans un plan parallèle à la fois à l'axe longitudinal du tibia et à l'axe longitudinal du fémur, lorsque ceux-ci ne sont pas parallèles, c'est-à-dire pas en position étendue du genou, d'une partie fémorale d'une prothèse du genou ; et
La figure 2 est une vue suivant la même coupe longitudinale d'une partie fémorale d'une prothèse posée sur un insert tibial, en position étendue du genou.

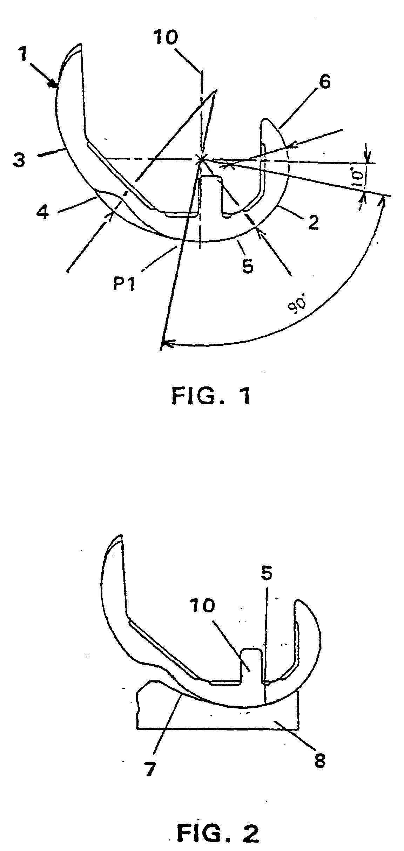

A la figure 1, la partie fémorale 1 d'une prothèse du genou est constituée de deux condyles 2 (puisqu'il s'agit d'une vue en coupe un seul apparaît à la figure 1) et d'une partie de support de trochlée 3 définissant un trajet 4 trochléen. Le trajet trochléen s'étend jusqu'au point P1, puis au-delà du point P1, du côté postérieur du genou, des arcs de cercle correspondent aux condyles. La surface extérieure des condyles est donc constituée, en coupe longitudinale, d'un premier arc de cercle 5 intermédiaire et d'un second arc de cercle 6 d'extrémité. L'arc de cercle 5 intermédiaire s'étend angulairement sur 90°. L'arc de cercle 6 d'extrémité s'étend angulairement sur 40°. Le rayon du cercle 6 d'extrémité est inférieur au rayon du cercle 5 intermédiaire. Le rayon du trajet trochléen (R1) est supérieur au rayon du cercle 5 intermédiaire.

Le rayon de courbure de la_surface 7 supérieure concave de l'insert tibial 8 est sensiblement identique aux rayons de courbure du cercle 5 intermédiaire.

On obtient ainsi une congruence sur le domaine de flexion de 0 à 90°, puis la congruence entre la partie fémorale diminue de sorte qu'un jeu apparaît entre eux, au-delà de 90°. Ce jeu va relâcher les contraintes sur les ligaments, notamment le ligament croisé postérieur. Le rapport du rayon intermédiaire sur le rayon d'extrémité est égal à 1,25. Suivant l'invention ce rapport doit être supérieur strictement à 1 et peut être égal même à 2 voire plus. Par rapport à l'axe vertical désigné par la référence numérique 10 au dessin, les condyles commencent 10° avant cet axe 10 c'est-à-dire sont décalés de 10° par rapport à la verticale.

## Revendications

1. Partie fémorale d'une prothèse du genou, de type à trois compartiments, comportant deux condyles (2) et une partie (3) de surface de trochlée, la partie fémorale étant destinée à être ancrée à l'extrémité distale d'un fémur, et la surface extérieure des condyles étant destinés à coopérer avec la surface (7) extérieure supérieure d'un insert tibial, en coupe longitudinale, la surface (3) de support de trochlée définissant un trajet trochléen suivant un premier arc de cercle, et toujours en coupe longitudinale, la surface extérieure des condyles (2) comportant au moins en partie un arc (5) de cercle intermédiaire, la surface extérieure des condyles (2), en coupe longitudinale, étant constituée d'une surface (5) intermédiaire et d'une surface (6) d'extrémité en arc de cercle toutes les deux, l'arc de cercle (5) intermédiaire s'étendant angulairement sur 80°à 90°, le rayon de courbure de l'arc (6) d'extrémité étant strictement inférieur au rayon de courbure de l'arc (5) intermédiaire, **caractérisée en ce que** l'arc de cercle (6) d'extrémité s'étend angulairement sur un angle compris entre 40° et 50°.

2. Partie fémorale suivant une prothèse du genou suivant la revendication 1, **caractérisée en ce que** le rapport de l'arc de cercle (5) intermédiaire sur l'arc de cercle (6) d'extrémité est compris entre 1 et 2,5 de préférence entre 1,2 et 1,8.

3. Prothèse du genou comportant une partie fémorale suivant la revendication 1 ou 2, et un insert tibial posé sur une plaque tibiale, l'insert tibial ayant une surface (7) concave supérieure coopérant avec la surface (7) extérieure des condyles, le rayon de courbure de la surface extérieure de l'insert étant sensiblement égal au rayon de courbure de l'arc de cercle (5) intermédiaire.

## Patentansprüche

1. Femurteil einer Knieprothese von der Art mit drei Segmenten, mit zwei Kondylen (2) und einem Trochlea-Oberflächenteil (3), wobei das Femurteil dazu bestimmt ist, am femen Ende eines Oberschenkelknochens verankert zu werden, und die Außenfläche der Kondyle dazu bestimmt ist, mit der oberen Außenfläche (7) eines Schienbeineinsatzes im Längsschnitt zusammenzuwirken, wobei die Trochlea-Stützfläche (3) einen Trochlea-Weg gemäß einem ersten Kreisbogen, immer noch im Längsschnitt, definiert, wobei die Außenfläche der Kondyle (2) zumindest zum Teil einen Zwischenkreisbogen (5) aufweist, wobei die Außenfläche der Kondyle (2) im Längsschnitt aus einer Zwischenfläche (5) und einer Endfläche (6), beide in Kreisbogenform, besteht, wobei der Zwischenkreisbogen (5) sich über eine Winkel von 80° bis 90° erstreckt, wobei der Krümmungsradius des Endbogens (6) strikt kleiner ist als der Krümmungsradius des Zwischenbogens (5), **dadurch gekennzeichnet, dass** der Endkreisbogen (6) sich über einen Winkel zwischen 40° und 50° erstreckt.

2. Femurteil e iner K nieprothese n ach Anspruch 1 , **dadurch gekennzeichnet, dass** das Verhältnis zwischen dem Zwischenkreisbogen (5) und dem Endkreisbogen (6) zwischen 1 und 2,5 und vorzugsweise zwischen 1,2 und 1,8 liegt.

3. Knieprothese, die ein Femurteil nach Anspruch 1 oder 2 und einen auf eine Schienbeinplatte aufgesetzten Schienbeineinsatz aufweist, wobei der Schienbeineinsatz eine obere konkave Fläche (7) aufweist, die mit der Außenfläche der Kondyle (2) zusammenwirkt, wobei der Krümmungsradius der Außenfläche des Einsatzes im Wesentlichen gleich dem Krümmungsradius des Zwischenkreisbogens (5) ist.

## Claims

1. A femoral part of a knee prosthetic of the type known as comprising three compartments, including two condyles **(2)** and a trochlea surface part **(3)** wherein the femoral part is to be anchored to the distal extremity of a femur and the external surface of the condyles is to co-operate with the upper external surface **(7)** of a tibia insert such that, in a longitudinal section, the trochlea support surface **(3)** defines a trochlean trajectory following a first circular arc and, still in a longitudinal section, the external surface of the condyles **(2)** includes at least partially an intermediary circular arc **(5)**, the external surface of the condyles **(2)**, in the longitudinal section, comprising an intermediary surface **(5)** and an extremity surface **(6)** both of which are a circular arc, with the intermediary circular arc **(5)** extending over an angle of 80º to 90º, the value of the curvature radius of the extremity arc **(6)** being strictly less than the value of the curvature radius of the intermediary arc **(5)**, **characterized in that** the extremity circular arc **(6)** extends over an angle comprised between 40º and 50º.

2. Femoral part of a knee prosthetic according to claim 1, **characterized in that** the ratio of the intermediary circular arc **(5)** over the extremity circular arc **(6)** stands between one and 2,5, preferably between 1,2 and 1,8.

3. A knee prosthetic including a femoral part according to claim 1 or 2, and a tibia insert laid onto a tibia plate, with said tibia insert having an upper concave surface **(7)** which co-operates with the external surface **(7)** of the condyles, as the curvature radius of the external surface of the insert is sensibly equal to the curvature radius of the intermediary circular arc **(5)**.
